# EUROPÄISCHE PATENTANMELDUNG

(11) **EP 1 502 620 A1**
(43) Veröffentlichungstag der Anmeldung: **02.02.2005**
(21) Anmeldenummer: 03014553.6
(22) Anmeldetag: 07.07.2003
(51) Int. Cl.: A61M 25/01, A61B 5/06, A61B 19/00, A61B 17/12

(54) **Verfahren und Vorrichtung zum Navigieren eines Objekts in einem Körper, insbesondere zu einem Aneurysma**

(71) Anmelder: BrainLAB AG, 85551 Kirchheim/Heimstetten (DE)
(72) Erfinder: Rainer Birkenbach, 85445 Aufkirchen (DE); Anke Weissenborn, 80805 München (DE); Dr. Ulrich Sure, 35037 Marburg (DE)
(74) Vertreter: Schwabe - Sandmair - Marx

(57) **Zusammenfassung**

Die Erfindung bezieht sich auf ein Verfahren zum Navigieren eines Objekts (7) in einem Körper (6), wobei der Verlauf von Blutgefäßen innerhalb des Körpers (6) durch Angiographie ermittelt wird, die Position des Objekts (7) durch einen mit dem Objekt (7) verbundenen Magnetfeldsensor (8) ermittelt wird und die relative Position des Objekts (7) im Körper (6) ermittelt wird, sowie auf eine Vorrichtung zum Navigieren eines Objekts (7) in einem Körper (6) mit einem Speicher, in welchem Daten zum Verlauf von Blutgefäßen innerhalb des Körpers (6) gespeichert werden können, einem Generator (4) zur Erzeugung eines magnetischen Feldes und einem Magnetfeldsensor (8), welcher mit dem Objekt (7) verbunden ist, um die Position des Objekts (7) innerhalb des Körpers (6) zu ermitteln.

## Beschreibung

Die vorliegende Erfindung bezieht sich auf ein Verfahren und eine Vorrichtung zum Navigieren oder Positionieren eines Objekts, wie z.B. eines Katheters, eines Drahtes oder einer aus Draht gebildeten Spule in einem Körper, insbesondere zu einem Aneurysma.

Das Einbringen eines Katheters in einen Körper zum Beispiel zum Einbringen eines Drahtes in ein Aneurysma ist im Stand der Technik bekannt und wird auch als "Coiling" bezeichnet. Dabei wird ein Führungsdraht oder Katheter von einer Zugangsstelle aus, wie z.B. einer Arterie im Arm, der Leiste oder der Halsschlagader, durch das Blutgefäßsystem zu dem gewünschten Ort, also zum Beispiel zum Aneurysma vorgeschoben, wobei die Blutbahnen als ein natürlicher Weg verwendet werden, durch welchen der Führungsdraht oder Katheter geführt werden kann. Am Zielort können durch den Katheter z.B. Stents, Spulen (Coils) oder andere Geräte positioniert werden. Ebenso ist es möglich aus dem Katheter bestimmte Substanzen wie z.B. Medikamente abzugeben, um diese gezielt an einen gewünschten Ort im Körper verabreichen zu können. Durch ein solches Verfahren kann z.B. eine an einer bestimmten Stelle verstopfte Ader mit einem geeigneten Werkzeug an der Spitze des Katheters geöffnet werden, ein Medikament an einen gewünschten Wirkungsort gebracht werden, ein minimal invasives Verfahren durchgeführt werden oder ein Gerät oder Instrument an einer bestimmten Stelle im Körper positioniert werden.

Hierzu ist es erforderlich, dass der Katheter bzw. die Spitze des Katheters von der Zugangsstelle aus durch den Körper insbesondere durch die Adern geführt wird, um an die gewünschte Stelle zu gelangen. Dabei muss zum Einen bekannt sein, wie die innere Struktur des Körpers aufgebaut ist, also wie z.B. die Blutbahnen verlaufen und weiterhin ist es erforderlich die momentane Position des Katheters oder der Katheterspitze zu kennen, um z.B. bei einer Abzweigung einer Ader die Katheterspitze in die gewünschte Richtung zu lenken.

Aus der US 6,522,909 B1 ist ein Verfahren zum Navigieren einer magnetischen Katheterspitze bekannt, wobei nach der Injektion eines Röntgenkontrastmittels ein Bild der Blutgefässe aufgenommen wird und anhand von weiteren in Echtzeit durchgeführten Röntgenaufnahmen der Katheter durch das Erzeugen eines gerichteten externen Magnetfeldes in eine gewünschte Richtung gelenkt wird.

Aus der US 6,332,089 B1 ist ein Verfahren zum Führen eines Katheters innerhalb des Körpers eines Patienten bekannt, wobei zunächst eine Sonde innerhalb des Körpers des Patienten an einer gewünschten Stelle positioniert wird und ein mit einer weiteren Sonde verbundenes Instrument basierend auf dem ermittelten relativen Lageverhältnis zwischen diesen Sonden zu dieser Stelle navigiert werden kann.

Die US 2 002/01 777 89 A1 beschreibt ein Vorschub-System zum Bewegen eines länglichen medizinischen Geräts innerhalb eines Körpers, welches durch ein magnetisches Navigationssystem gesteuert werden kann, so dass ein Chirurg einen Katheter in einem Körper steuern kann, ohne sich der für die Navigation benötigten Röntgenstrahlung auszusetzen.

Weitere Verfahren und Vorrichtungen für invasive Eingriffe sind aus der US 2 002/00 165 42 A1, US 2 001/00 386 83 A1, US 2 002/01 004 86 A1, US 6,330,467 B1, US 2 003/00 740 11 A1, und US 6,216,028 B1 bekannt.

Es ist eine Aufgabe der vorliegenden Erfindung ein Verfahren und eine Vorrichtung zum Navigieren oder auch Positionieren eines Objekts, wie z.B. eines Katheters, in einem Körper vorzuschlagen, welche einfach angewendet werden können und die Gesundheit eines Patienten möglichst wenig belasten.

Diese Aufgabe wird durch das Verfahren und die Vorrichtung wie in den unabhängigen Ansprüchen definiert gelöst. Vorteilhafte Ausführungsformen ergeben sich aus den Unteransprüchen.

Bei dem erfindungsgemäßen Verfahren zum Navigieren bzw. Führen oder Positionieren eines Objekts, wie z.B. eines Katheters, eines Führungsdrahtes oder einer Spitze dieser Elemente in einem Körper wird zunächst der Verlauf der Blutgefäße in dem Körper durch Injektion eines geeigneten Mittels, wie z.B. eines Röntgenkontrastmittels, und anschließender Erfassung von Bilddaten ermittelt. Solche als Angiographie bezeichneten Gefäßdarstellungen durch Injektion z.B. eines Röntgenkontrastmittels und anschließende Anfertigung schneller, programmierter Aufnahmeserien oder Angiogramme sind im Stand der Technik bekannt und z.B. in der US 6,522,909 B1 beschrieben, deren diesbezügliche Lehre in diese Anmeldung aufgenommen wird. Es kann z.B. eine Arteriographie, z.B. als Karotis-, Aorto- oder Angiokardiographie oder eine Phlebo- oder Lymphangiographie durchgeführt werden, um den Verlauf der Blutbahnen in einem bestimmten Bereich des Körpers zu ermitteln. Der so ermittelte Verlauf der Blutbahnen oder -gefäße wird gespeichert und die Position des in den Körper einzubringenden Objekts, wie z.B. des Katheters oder der Katheterspitze, wird erfindungsgemäß durch ein magnetisches Verfahren ermittelt. Beispielsweise können an dem einzubringenden Objekt oder an dem Katheter bzw. an dessen Spitze ein oder mehrere Magnetfeldsensoren, wie zum Beispiel eine oder mehrere Spulen oder auch ein oder mehrere Magneten angebracht sein, welche es ermöglichen, dass die Position des Objekts oder des Katheters durch ein z.B. von einem externen Generator erzeugtes Magnetfeld oder durch Sensorelemente ermittelt werden kann. Magnetfeldsensoren können zum Beispiel in verschiedenen Ausrichtungen vorgesehen sein, um zum Beispiel x, y und z-Komponenten eines Magnetfeldes zu erfassen.

Verfahren zur magnetischen Positionserkennung oder zur magnetischen Navigation sind z.B. aus der europäischen Patentanmeldung mit der Nummer EP 02 017 736.6 der Anmelderin, der US 6,332,089 B1 oder der US 6,216,028 B1 bekannt, deren technische Lehren bezüglich der Ermittlung einer Position unter Verwendung eines Magnetfeldes bzw. bezüglich des magnetischen Trackings in diese Anmeldung aufgenommen werden. Ist weiterhin die Position des Körpers bekannt, z.B. dadurch, dass an dem Körper optische oder magnetische aktive oder passive Marker, wie z.B. eine oder mehrere Spulen oder Magnete, angebracht sind, so kann aus den vorher ermittelten Daten zum Verlauf der Blutgefäße im Körper in Verbindung mit den durch ein Magnetfeld ermittelten Positionsdaten des Magnetfeldsensors oder Magnets bestimmt werden, wie die Lage des Magnetfeldsensors oder Magnets bzw. des damit verbundenen Objekts in dem Körper und insbesondere in den Blutgefäßen ist, so dass es möglich ist, das Objekt durch die Blutgefäße zu einer gewünschten Stelle zu steuern oder zu navigieren.

Durch das erfindungsgemäße Verfahren ist es nicht mehr erforderlich, dass zum Ermitteln der Position des zu navigierenden Objekts Röntgenstrahlen verwendet werden müssen. Die erfindungsgemäße Positionsbestimmung kann durch einen Magnetfeldsensor oder Magneten erfolgen, wodurch die Strahlenbelastung für den Patienten verringert wird und auch keine Gefahr durch ionisierende Strahlung für behandelnde Personen mehr besteht. Demzufolge ist es möglich, die Strahlenbelastung eines Patienten beim Navigieren eines Katheters auf ein Minimum zu reduzieren, z.B. auf die Durchführung einer einzigen oder weniger Angiographien.

In einer bevorzugten Ausführungsform des erfindungsgemäßen Verfahrens kann zuerst ein Gesamtbild des Blutgefäßsystems eines Patienten zum Beispiel von einer Zugangsstelle bis zu einem Zielort ermittelt werden, indem z.B. ein Röntgenkontrastmittel gespritzt wird und aus der Verteilung des Röntgenkontrastmittels in den Blutbahnen der gesamte Verlauf der Blutgefäße durch eine einzelne Aufnahme oder eine Zusammensetzung mehrerer Aufnahmen ermittelt wird, um Informationen zum Verlauf der Blutgefäße von einer Einbringstelle des Objekts bis zum gewünschten Zielort zu haben. Eine solche Landkarte aller interessierenden Blutbahnen kann abgespeichert werden und kann als Grundlage für das nachfolgende magnetische Tracking des einzubringenden Objekts dienen.

Alternativ oder ergänzend kann auch nur für einen bestimmten Teil der Blutgefäße eine Angiopraphie durchgeführt werden, um z.B. Daten zur aktuellen Umgebung des einzubringenden Objekts zu haben, wobei nach einer Weiterbewegung des einzubringenden Objekts wiederum eine lokal begrenzet Angiographie durchgeführt wird, um die nächsten Bilddaten zum Verlauf der Blutgefäße für die Navigation des Objekts zu erhalten.

Es können zur Ermittlung des Verlaufs der Blutgefäße prinzipiell alle bekannten Angiographie-Verfahren durchgeführt werden, wie z.B. eine Computertomographie-Angiographie (CTA), eine Kernspinresonanz-Angiographie (MRA) oder eine digitale Subtraktions-Angiographie (DSA).

Das in den Körper einzubringende Objekt ist bevorzugt ein Katheter oder ein Führungsdraht, wobei mit einem solchen Katheter oder Führungsdraht auch andere Objekte oder Instrumente an einen Zielort, wie z.B. ein Aneurysma, gebracht werden können und dort z.B. von dem Katheter abgekoppelt werden. Beispielsweise können Stents oder Ballone zu einem Aneurysma gebracht werden oder es kann aus einem Katheter z.B. ein Draht ausgeschoben werden, welcher in ein Aneurysma eingebracht wird und sich zu einer Spule oder Wicklung formt, um das Aneurysma zu verschließen oder zu verstopfen, was als coiling bezeichnet wird. Das Einbringen solcher Coils in ein Aneurysma ist aus der US 6,522,909 B1 bekannt, deren diesbezügliche Lehre in diese Anmeldung aufgenommen wird. Vorteilhaft kann die Ablösung eines aus einem Katheter ausgeschobenen und gewickelten bzw. eingedrehten Drahtes durch Anlegen eines Stromes bewirkt werden, welcher dazu führt, dass der Draht an einer bestimmten Stelle durchgeschmolzen wird und somit z.B. der noch in dem Katheter vorhandene Draht zusammen mit dem Katheter wieder durch das Blutgefäßsystem zurückgezogen wird, wobei die eingebrachten Coils im Aneurysma verbleiben.

Vorteilhaft ist ein Anzeigeelement, wie z.B. ein Bildschirm vorgesehen, auf welchem der durch Angiographie ermittelte Verlauf von Blutgefäßen dargestellt werden kann und auf welchem weiterhin die Position eines magnetischen Sensors, eines Magneten oder eines mit diesem Sensor oder Magneten verbundenen Objekts, wie z.B. eines Katheters, gezeigt werden kann, so dass erkannt werden kann, wie der magnetische Sensor oder das mit diesem verbundene Objekt in den Blutgefäßen liegt, um das Objekt durch die Blutgefäße hindurch steuern zu können.

Weiterhin bezieht sich die Erfindung auf ein Computerprogramm, welches, wenn es in einem Computer geladen ist bzw. wird oder auf einem Computer läuft, einen oder mehrere der oben beschriebenen Verfahrensschritte durchführt. Des Weiteren bezieht sich die Erfindung auf ein Programmspeichermedium oder ein Computerprogrammprodukt mit einem solchen Programm.

Gemäß einem weiteren Aspekt bezieht sich die Erfindung auf eine Vorrichtung zum Navigieren oder Positionieren eines Objekts in einem Körper mit einer Vorrichtung, welche Daten zum Verlauf von Blutgefäßen speichert. Solche Daten können beispielsweise durch eine Vorrichtung zur Durchführung einer Angiographie ermittelt werden. Erfindungsgemäß ist ein magnetisches Positionserfassungssystem oder magnetisches Trackingsystem, wie beispielsweise in der europäischen Patentanmeldung mit der Anmeldenummer EP 02 017 736.6 der Anmelderin oder im oben zitierten Stand der Technik beschrieben vorgesehen, um die Position eines in den Körper einzubringenden Objekts, welches bevorzugt mit mindestens einem magnetischen Sensor oder einem Magneten verbunden ist, zu ermitteln und um die relative Lage des einzubringenden Objekts im Gefäßsystem des Körpers zu bestimmen. Ein magnetisches Trackingsystem kann zum Beispiel einen Magnetfeldgenerator aufweisen, welcher ein Magnetfeld erzeugt, anhand dessen die Position eines Magnetfeldsensors, wie zum Beispiel einer oder mehrerer Spulen, ermittelt werden kann. Ebenso können Magnetfeldsensoren vorgesehen sein, welche die Position eines Magneten ermitteln können.

Bevorzugt weist die erfindungsgemäße Vorrichtung auch eine Angiographie-Vorrichtung auf, mit welcher z.B. der Verlauf von Blutgefäßsystemen von einem Einbringpunkt des Objekts bis zu einem gewünschten Zielpunkt innerhalb des Körpers ermittelt werden kann, oder welches es ermöglicht, während eines Eingriffs, also Intra-OP, eine Angiographie eines bestimmten Bereichs des Körpers durchzuführen, um Daten zum Verlauf der Blutgefäße zu ermitteln. Die so ermittelten Daten können zum Speicher der oben beschriebenen Vorrichtung übermittelt werden.

Vorteilhaft ist ein Bildschirm vorgesehen, auf welchem der durch Angiographie ermittelte Verlauf von Blutgefäßen zusammen mit der Position des einzubringenden Objekts relativ zu den Blutgefäßen dargestellt werden kann.

Das einzubringende Objekt ist bevorzugt ein Führungsdraht oder ein Katheter, mit welchem z.B. Coils, Stents oder auch bestimmte Medikamente an einen Zielort innerhalb des Körpers gebracht werden können.

Die Erfindung wird nachfolgend anhand bevorzugter Ausführungsformen beschrieben werden. Es zeigen:
- Fig. 1: eine erfindungsgemäße Vorrichtung zum Navigieren eines Katheters in einen Körper;
- Fig. 2: eine Ausführungsform eines erfindungsgemäß verwendbaren Katheters;
- Fig. 3: ein Blutgefäßsystem, durch welches ein Katheter bewegt wird;
- Fig. 4: ein Blutgefäß mit einem Aneurysma, in welches ein Draht zum Verschließen des Aneurysmas eingebracht wird;
- Fig. 5: das in Fig. 4 gezeigte Aneurysma nach dem Einbringen des Drahtes; und
- Fig. 6: ein Blutgefäß mit einem eingebrachten Stent und eingebrachten Coils.

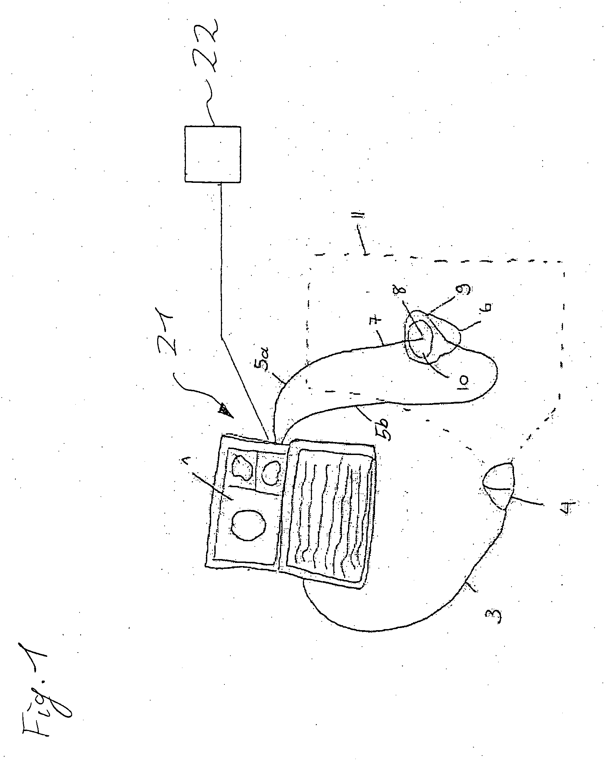

Figur 1 zeigt schematisch eine erfindungsgemäße Vorrichtung, mit welcher das erfindungsgemäße Verfahren durchgeführt werden kann und zeigt einen Computer 21, in welchem durch eine Angiographie-Vorrichtung 22 ermittelte Daten zum Verlauf von Blutgefäßen eines Körpers gespeichert sind. Mit dem Computer 21 ist ein Generator 4 zur Erzeugung eines magnetischen Feldes 11 über eine Leitung 3 verbunden. Innerhalb des von dem Generator 4 erzeugten magnetischen Feldes 11 befindet sich ein Objekt oder Körper, wie zum Beispiel ein Kopf 6, in welchen ein Katheter 7 eingebracht und in diesem navigiert werden soll. Auf einem Bildschirm 1 ist die Struktur von Blutgefäßen innerhalb des Körpers 6 dargestellt. Durch einen fest an dem Körper 6 angebrachten Referenz-Sensor 9, welcher der Bewegung des Körpers 6 folgt, kann die räumliche Lage des Körpers 6 ermittelt werden.

An der Spitze des Katheters 7 ist eine Spule 8 angebracht, welche als ein Magnetfeld-Sensor wirkt, so dass von dem Computer 21, welcher über Leitungen 5a und 5b mit den Magnetfeld-Sensoren 8 und 9 verbunden ist, die relative Lage des Sensors bzw. der Spule 8 und damit der Spitze des Katheters 7 im Körper 6 ermittelt werden kann. Somit kann auf dem Bildschirm 1 die durch ein magnetisches Tracking ermittelte Position der Katheterspitze innerhalb des durch Angiographie ermittelten Verlaufs der Blutgefäße dargestellt werden, so dass der Katheter 7 durch bekannte Steuerbewegungen, wie zum Beispiel das Biegen der Katheterspitze in eine gewünschte Richtung, durch das Blutgefäßsystem hindurch bis zu einer gewünschten Stelle zum Beispiel innerhalb des schematisch gezeigten Gehirns 10 vorgeschoben werden kann.

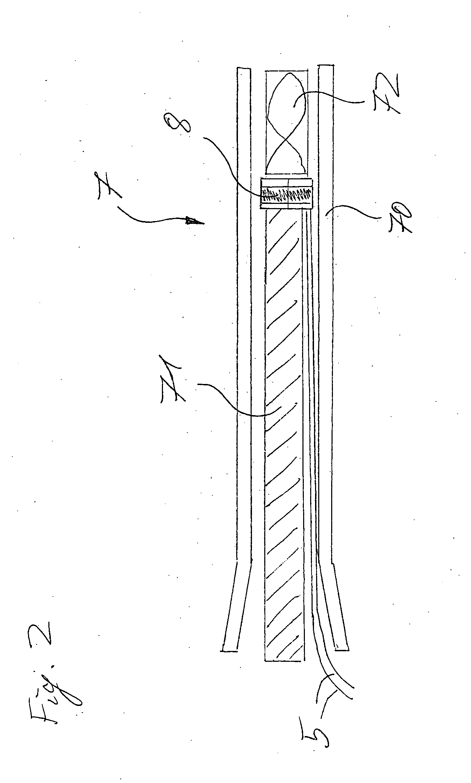

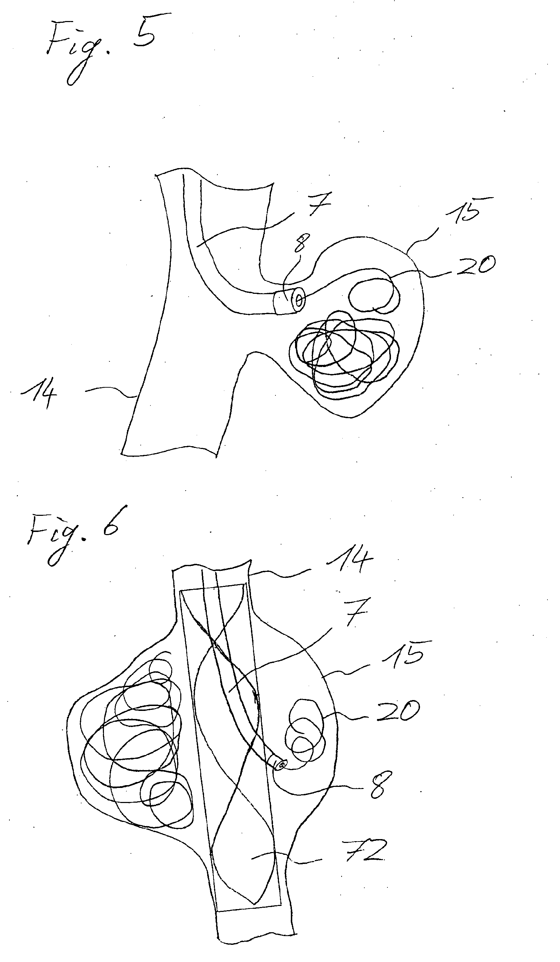

Figur 2 zeigt schematisch eine Ausführungsform eines Katheters 7 mit einem länglichen Schlauch oder einer Hülse 70, in deren Inneren ein Draht 71 in axialer Richtung des Schlauchs oder der Hülse 70 verschiebbar angeordnet ist. An der Spitze des Drahtes 71 ist ein Magnetfeldsensor 8 angebracht, um die Position der Drahtspitze und damit der Katheterspitze zu ermitteln. An dem Sensor 8 sind elektrische Leitungen 5 befestigt, welche mit dem in Figur 1 gezeigten Computer 21 verbunden werden können. Ein solcher Katheter kann durch magnetisches Tracking oder magnetische Navigation von einer Eingangsstelle aus durch ein Blutgefäßsystem hindurch zu einer gewünschten Stelle innerhalb eines Körpers bewegt werden, um dort zum Beispiel einen in Figur 6 gezeigten Stent 72 einzubringen.

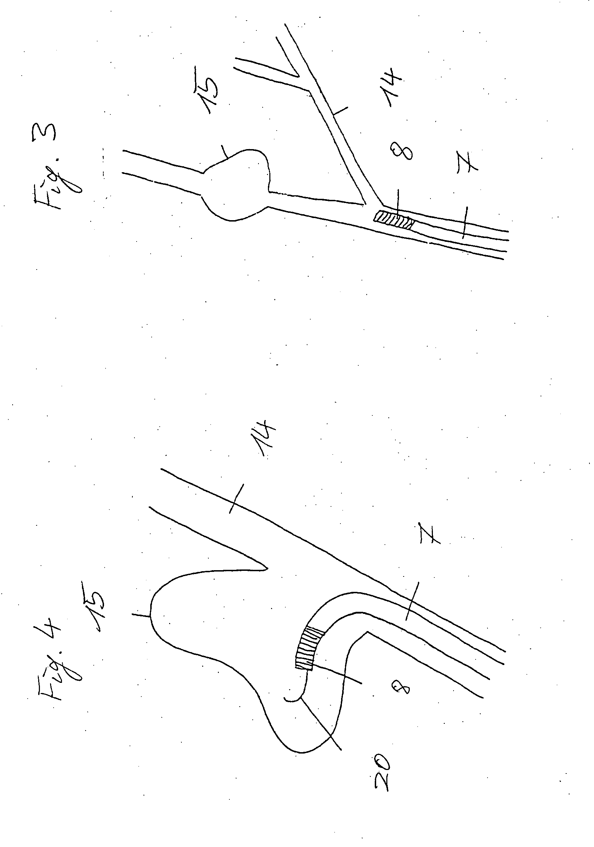

Figur 3 zeigt schematisch ein Blutgefäßsystem 14 mit einer weiteren Ausführungsform eines Führungsdrahtes oder Katheters 7, an dessen Spitze eine Spule 8 als Magnetfeldsensor angebracht ist. Die Position des Führungsdrahtes oder Katheters 7 wird über das von dem Generator 4 (Figur 1) erzeugte Magnetfeld und die Spule 8 ermittelt und kann auf dem Bildschirm 1 relativ zu dem Verlauf der Blutgefäße 14 dargestellt werden, so dass der Führungsdraht oder Katheter 7 zu dem gezeigten Aneurysma 15 navigiert werden kann.

Figur 4 zeigt den zu dem Aneurysma 15 navigierten Katheter 8, dessen Ende in das Aneurysma 15 hinein bewegt wird. Wird der Katheter 8 in der in Figur 4 gezeigten Position gehalten, so kann aus dem Katheter ein Draht 20 ausgeschoben werden, welcher sich zu einer Spule wickelt, um das Aneurysma 15 auszufüllen.

In Figur 5 ist ein durch einen gewickelten Draht zum Teil gefülltes Aneurysma 15 gezeigt, wobei der Draht bereits abgetrennt wurde, um noch einen weiteren Bereich des Aneurysmas ausfüllen zu können.

Figur 6 zeigt ein Blutgefäß, in welches ein Stent 72 im Bereich eines Aneurysmas 15 eingebracht wurde und bei welchem Drähte 20 zum Füllen und Verschließen des Aneurysmas 15 außerhalb des Stents 72 in das Aneurysma 15 eingeschoben wurden.

## Patentansprüche

1. Verfahren zum Navigieren eines Objekts (7) in einem Körper (6), insbesondere zu einem Aneurysma, wobei der Verlauf von Blutgefäßen (14) innerhalb des Körpers (6) durch Angiographie ermittelt wird, die räumliche Position des Objekts (7) durch einen mit dem Objekt (7) verbundenen Magnetfeldsensor (8) oder Magnet ermittelt wird und aus dem Verlauf der Blutgefäße (14) und der räumlichen Position des Objekts (7) die relative Position des Objekts (7) im Körper (6), insbesondere in den Blutgefäßen (14) ermittelt wird.

2. Verfahren nach dem vorhergehenden Anspruch, wobei der durch Angiographie ermittelte Verlauf der Blutgefäße gespeichert wird.

3. Verfahren nach den vorhergehenden Ansprüchen, wobei während des Navigierens des Objektes (7) in dem Körper (6) mindestens eine weitere Angiographie durchgeführt wird.

4. Verfahren nach einem der vorhergehenden Ansprüche, wobei die relative Position des Objektes (7) im Blutgefäßsystem des Körpers (6) angezeigt wird.

5. Computerprogramm, welches, wenn es in einem Computer geladen ist oder auf einem Computer läuft, ein Verfahren nach einem der vorhergehenden Ansprüche durchführt.

6. Programmspeichermedium oder Computerprogrammprodukt mit dem Programm nach dem vorhergehenden Anspruch.

7. Vorrichtung zum Navigieren eines Objekts (7) in einem Körper (6), insbesondere zu einem Aneurysma mit einem Speicher, in welchem Daten zum Verlauf von Blutgefäßen innerhalb des Körpers (6) gespeichert werden können, einem Generator (4) zur Erzeugung eines magnetischen Feldes und einem Magnetfeldsensor (8), welcher mit dem Objekt (7) verbunden ist, um die Position des Objekts (7) innerhalb des Körpers (6) zu ermitteln.

8. Vorrichtung nach dem vorhergehenden Anspruch mit einer Vorrichtung (22) zur Durchführung einer Angiographie.

9. Vorrichtung nach einem der zwei vorhergehenden Ansprüche mit einer Anzeige (1), auf welcher der Verlauf von Blutgefäßen und die relative Position des Objektes (7) dargestellt werden kann.

10. Vorrichtung nach einem der drei vorhergehenden Ansprüche, wobei das Objekt (7) ein Katheter oder ein Führungsdraht oder zur Einbringung von coils geeignet ist.
